# EUROPEAN PATENT SPECIFICATION

(11) **EP 0 902 026 B1**
(45) Date of publication and mention of the grant of the patent: **12.04.2006**
(21) Application number: 98307366.9
(22) Date of filing: 11.09.1998
(51) Int. Cl.: C07D 401/14, C07D 401/12, C07D 409/14, C07D 213/64, A01N 43/54, A01N 43/44, A01N 43/56, C07D 401/04

(54) **Herbicidal 2-azinyl-6-aryloxypyr(mi)dines**
2-Azinyl-6-aryloxypyri(mi)dien-Derivate als Herbizide
2-azinyl-6-aryloxypyri(mi)dines comme herbicides

(30) Priority: 12.09.1997 US 928534
(43) Date of publication of application: 17.03.1999
(73) Proprietor: BASF Aktiengesellschaft, 67056 Ludwigshafen (DE)
(72) Inventor: Scheiblich, Stefan, 55128 Mainz (DE); Kleemann, Axel, 63454 Hanau (DE); Maier, Thomas, 55127 Mainz (DE); Baltruschat, Helmut Siegfried, 55444 Schweppenhausen (DE)
(74) Representative: Langfinger, Klaus Dieter

(56) References cited:
- EP-A- 0 407 899
- EP-A- 0 723 960
- WO-A-92/10490
- DE-A- 4 029 649
- US-A- 4 927 827
- US-A- 5 022 915
- CHEMICAL ABSTRACTS, vol. 116, no. 5, 3 February 1992 Columbus, Ohio, US; abstract no. 36253n, OKAMOTO, TOMOKO ET AL: "Plant growth regulators containing pyrimidine derivatives" XP002088962 -& CHEMICAL ABSTRACTS 13TH COLLECTIVE CHEMICAL SUBSTANCE INDEX. BOOK 52:, page 932 XP002088961 COLUMBUS US & JP 03 169802 A (SUMITOMO CHEMICAL CO., LTD., JAPAN)

## Description

### BACKGROUND OF THE INVENTION

This invention relates to certain novel 2-azinyl-6-aryloxypyri(mi)dines, to the preparation of such compounds, to herbicidal compositions containing such compounds, and to a method of combating undesired plant growth using such compounds.

Pyridines, pyrimidines and their derivatives have many uses in the pharmaceutical area as well as in agriculture (herbicides, fungicides, acaricides, anthelmintics, bird repellents), as reagents, intermediates and chemicals for the polymer and textile industry.

The broad generic formula of the International patent application WO 96/06096 embraces 2-azolyl-5-aryloxypyridines.

Similar 2-aryl-5-aryloxypyri(mi)dines are disclosed by EP 0 723 960. The European patent application EP 0 451 585 discloses herbicidal 2-heteroaryl-6-arylpyridines and the US patent no. US 5,022,915 discloses herbicidal 2-heteroaryl-6-arylpyrimidines. However, there is no disclosure of 2-azinyl-6-aryloxypyri(mi)dines in any of these documents.

Although many of the known compounds show considerable activity against various weeds, they are not completely satisfying with regard to their selectivity or because of their persistence.

The broad generic formula of EP 0 431 424 embraces fungicidal 2-aryloxy-6-pyrimid-2'-ylpyridines.
The German patent application DE 40 29 649 discloses fungicidal 4-phenyloxy- and 4-phenylthio-2-(4-ethoxypyrid-2-yl)-6-methylpyrimidines. There is no disclosure of any herbicidal activity in these documents.
The compounds according to the present invention combine high herbicidal activity with good selectivity and a desirable rate of degradation in soil.
The present invention provides novel compounds of the general formula I 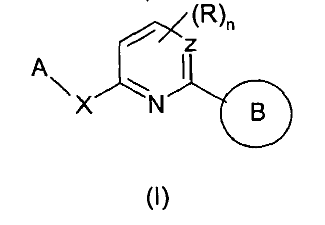 wherein
- A: represents a phenyl, pyridyl, pyrazolyl or thienyl group, substituted by one or more of the same or different substituents selected from halogen atoms, C₁-C₄-alkyl groups, C₁-C₄-alkoxy groups, cyano groups, C₁-C₄-haloalkyl groups, C₁-C₄₋haloalkoxy groups, C₁-C₄-alkylthio groups, C₁-C₄-haloalkylthio groups and SF₅ groups, and wherein A has a substituent in the meta-position relative to the point of attachment;
- B: represents an optionally substituted 6-membered nitrogen-containing heteroaromatic group, in which the optional substituents are one or more, same or different, of the following: halogen, nitro, cyano, amino, hydroxy, phenoxy, C₁₋₄alkyl, C₁₋₄alkoxy, C₁₋₄haloalkyl, C₁₋₄haloalkenyl, C₁₋₄haloalkoxy, C₁₋₄haloalkylthio, C₁₋₄alkylsulfonyl and halosulfanyl;
- R: represents a halogen atom or an optionally substituted alkyl, alkenyl, alkynyl, alkoxy, alkoxyalkyl, alkoxyalkoxy group; or a haloalkyl, haloalkoxy, cyano, nitro group; or -S(O)ₚ-R⁵, in which p is 0, 1 or 2, and in which R⁵ represents an alkyl or haloalkyl group; or -NR⁶R⁷, in which R⁶ and R⁷ independently represent a hydrogen atom, an alkyl, alkenyl, aralkyl or aryl group; or R⁸O-CY-, in which R⁸ represents an alkyl group, and Y represents O or S;
in which the optional substituents for an optionally substituted alkyl group or alkyl part are selected from phenyl, halogen atoms, nitro, cyano, hydroxyl, C₁-C₄-alkoxy, C₁-C₄-haloalkoxy and C₁-C₄-alkoxycarbonyl groups;
wherein the alkyl, alkenyl and alkynyl moieties contain 1 to 6 carbon atoms; the alkyl parts of haloalkyl, haloalkoxy, alkylthio or alkoxy group contain 1 to 4 carbon atoms and the alkoxyalkyl and alkoxyalkoxy group contain up to 6 carbon atoms;
- X: represents an oxygen or a sulfur atom,
- Z: represents CH or N, and
- n: represents an integer from 0 to 3,
with the provisos that
B represents an optionally substituted pyridine group when Z denotes CH.

The new compounds show an excellent selective herbicidal activity in certain crops, such as maize and rice, and degrade well in soil.

It is an object of the present invention to provide novel herbicidal compounds.

It is another object of the invention to provide methods for controlling undesired plant growth by contacting said plants with a herbicidally effective amount of the new compounds.

It is another object of the invention to provide selective herbicidal compositions containing the new compounds as active ingredients.

It is another object of the invention to provide new processes for the preparation of the new compounds.

These and other objects and features of the invention will become more apparent from the detailed description set forth hereinbelow.

It has surprisingly been found that the novel compounds of formula I, in which R, A, B, X, Z and n have the meaning given above, show excellent herbicidal activity against a broad range of weeds.

The expression "pyri(mi)dine" as used hereinbefore or hereinbelow includes both pyridine and pyrimidine moieties. The expression "azinyl" as used hereinbefore or hereinbelow includes 6-membered heteroaryl groups containing at least one nitrogen atom.

An aryl group as substituent or part of substituents is suitably an optionally substituted phenyl group.

A represents a phenyl, pyridyl, thienyl or pyrazolyl group being substituted by one or more of the same or different substituents selected from halogen atoms, alkyl groups, alkoxy groups, cyano groups, haloalkyl groups, haloalkoxy groups, alkylthio groups, haloalkylthio groups and SF₅ groups, and has a substituent in the meta-position relative to the point of attachment; more preferably A is meta-substituted by a fluorine or chlorine atom, or a trifluoromethyl, trifluoromethoxy or difluoromethoxy group. If A represents a thienyl group, it may be attached in the 2- or 3-position with respect to the sulfur atom. 3-thienyl groups are preferred.

B preferably represents a pyridyl group being substituted by one or more of the same or different substituents selected from halogen atoms, alkyl groups, alkoxy groups, cyano groups, nitro groups, haloalkyl groups, haloalkoxy groups alkylthio groups, haloalkylthio groups and SF₅ groups, in particular wherein the pyridyl group is attached to the central pyri(mi)dine ring in its 3- or 4-position and is substituted in the 6-position by a substituent selected from halogen atoms and haloalkyl or haloalkoxy groups.

Generally, if any of the above mentioned moieties comprises an alkyl, alkenyl or alkynyl group, such groups, unless otherwise specified, may be linear or branched and may contain 1 to 6, preferably 1 to 4, carbon atoms. Examples of such groups are methyl, ethyl, propyl, vinyl, allyl, propargyl, isopropyl, butyl, isobutyl and tertiary-butyl groups. The alkyl portion of a haloalkyl, haloalkoxy, haloalkylthio, alkylthio or alkoxy group suitably has from 1 to 4 carbon atoms, preferably 1 or 2 carbon atoms. The number of carbon atoms in the alkoxyalkyl, alkoxyalkoxy or dialkoxyalkyl groups is up to 6, preferably up to 4, e.g. methoxymethyl, methoxymethoxy, methoxyethyl, ethoxymethyl, ethoxyethoxy, dimethoxymethyl.

"Halogen" means a fluorine, chlorine, bromine or iodine atom, preferably fluorine, chlorine or bromine. Haloalkyl moieties of any groups within the definitions used herein and as such can contain one or more halogen atoms. Haloalkyl, haloalkoxy and haloalkylthio are preferably mono-, di-, tri- or perfluoroalkyl, -alkoxy and -alkylthio, especially trifluoromethyl, pentafluoroethyl, trifluoromethoxy, difluoromethoxy, difluoromethylthio, trifluoromethylthio or 2,2,2-trifluoroethoxy groups.

When any groups are designated as being optionally substituted, the optional substituent groups may be any of those customarily employed in the modification and/or development of pesticidal compounds and are especially substituents that maintain or enhance the herbicidal activity associated with the compounds of the present invention, or influence persistence of action, soil or plant penetration, or any other desirable property of such herbicidal compounds.

There may be one or more of the same or different substituents present in each part of the molecules. In relation to moieties defined above as comprising an optionally substituted alkyl group, including alkyl parts of haloalkyl, alkoxy, alkylthio, haloalkoxy, alkylamino and dialkylamino groups, specific examples of such substituents include phenyl, halogen atoms, nitro, cyano, hydroxyl, C₁₋₄-alkoxy, C₁₋₄-haloalkoxy and C₁₋₄-alkoxycarbonyl groups.

In relation to moieties defined above as comprising an optionally substituted aryl or heteroaryl group, optional substituents include halogen, especially fluorine, chlorine and bromine atoms, and nitro, cyano, amino, hydroxy, phenoxy, C₁₋₄-alkyl, C₁₋₄-alkoxy, C₁₋₄-haloalkyl, C₁₋₄-haloalkenyl, C₁₋₄₋haloalkoxy, C₁₋₄-haloalkylthio, C₁₋₄-alkylsulfonyl and halosulfanyl groups such as SF₅. In the case of phenyl-groups 1 to 5 substituents may suitably be employed, in the case of thienyl-groups 1 to 3 substituents may suitably be employed, 1 or 2 substituents being preferred.

Typically haloalkyl, haloalkoxy and haloalkylthio groups are trifluoromethyl, pentafluoroethyl, trifluoromethoxy, difluoromethoxy, 2,2,2-trifluoroethoxy and trifluoromethylthio groups.

In other preferred embodiments of formula I, A represents a group of formula a, b, c or d: 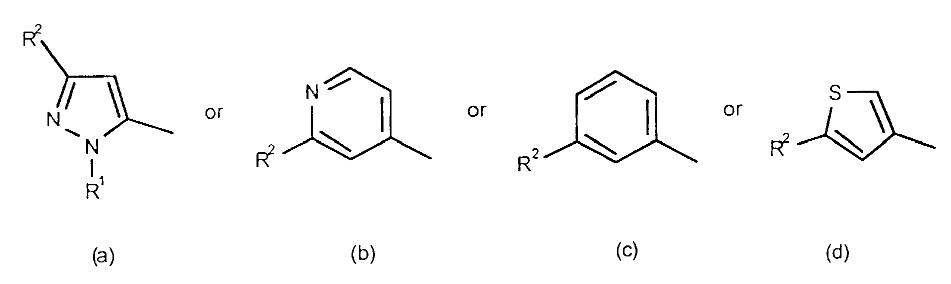 wherein R¹ is C₁₋₃ alkyl and R² is C₁₋₄ alkyl, C₁₋₃ haloalkyl, a halogen atom, cyano, C₁₋₃ haloalkoxy or C₁₋₃ haloalkylthio; while B preferably represents a group of formula e or f: 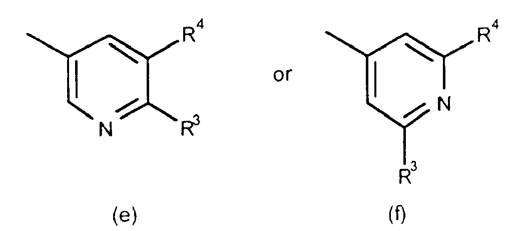 wherein R³ is C₁₋₄ alkyl, C₁₋₃ haloalkyl, a halogen atom, cyano, C₁₋₃ haloalkoxy or C₁₋₃ haloalkylthio and R⁴ is hydrogen, halogen or C₁₋₃ alkyl. Particularly preferred are the compounds of formula IA and IB: 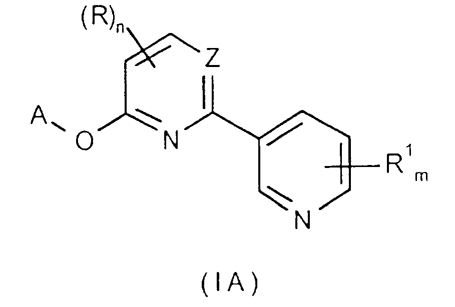 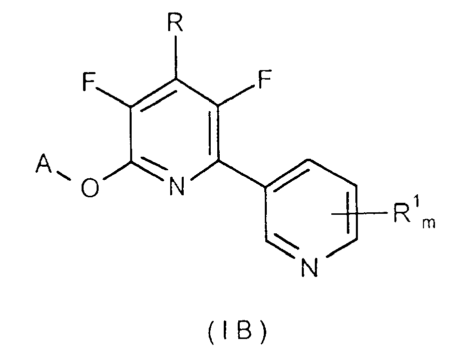 wherein A represents 3-trifluoromethylphenyl, 2-chloropyrid-4-yl, 2-trifluoromethylpyrid-4-yl, 2-difluoromethoxypyrid-4-yl, 2-trifluoromethylthien-4-yl or 1-methyl-3-trifluoromethylpyrazol-5-yl, Z and R have the meaning given above; R¹ each independently represent a hydrogen atom or a fluorine atom, one or two of them also a chlorine or bromine or a trifluoromethyl, difluoromethoxy or a cyano group, one of them can further be a C₁-C₄-alkyl group, particularly tert-butyl, at least one of R¹ being different from hydrogen, m is an integer selected from 1 to 4, and n is an integer selected from 0 to 2, preferably 0 or 1.

Suitably, R represents a halogen atom or an optionally substituted alkyl, alkoxy group.

Preferably, R represents an optionally substituted C₁₋₆ alkyl group or C₁₋₆ alkoxy group which is unsubstituted, or substituted by one or more moieties independently selected from halogen atoms. In particular R denotes a fluorine or chlorine atom or a methyl, ethyl or methoxy group.

The invention is exemplified by the following specific compounds:
6-methyl-4-(1'-methyl-3'-trifluoromethylpyrazol-5'-yloxy)-2-(6"-trifluoromethylpyrid-3"-yl)pyrimidine,
6-methyl-4-(1'-methyl-3'-trifluoromethylpyrazol-5'-yloxy)-2-(6"-chloropyrid-3"-yl)pyrimidine,
6-methyl-4-(3'-trifluoromethylphenyloxy)-2-(6"-trifluoromethyl-pyrid-3"-yl)pyrimidine,
6-methyl-4-(3'-trifluoromethylphenyloxy)-2-(6"-chloropyrid-3"-yl)pyrimidine, 5-methyl-4-(3'-trifluoromethylphenyloxy)-2-(6"-trifluoromethylpyrid-3"-yl)pyrimidine,
5-methyl-4-(1'-methyl-3'-trifluoromethylpyrazol-5'-yloxy)-2-(6"-trifluoromethylpyrid-3"-yl)pyrimidine; and
5-methyl-4-(6'-trifluoromethylpyrid-3-yloxy)-2-(6"-trifluoromethylpyrid-3"-yl)pyrimidine.

The compounds of this invention may be oils, gums, or, predominantly, crystalline solid materials. They can be used in agriculture or related fields for the control of undesired plants. The compounds of general formula I according to the invention possess a high herbicidal activity within a wide concentration range and at low dosages, and may readily be used in agriculture, especially for the selective control of undesired plants such as *Alopecurus myosuroides, Echinochloa crus-galli, Setaria viridis, Galium aparine, Stellaria media, Veronica persica, Lamium purpureum, Viola arvensis, Abutilon theophrasti, lpomoea purpurea* and *Amaranthus retroflexus* by pre- and post-emergence application, and particularly in certain crops, such as maize and rice.

The compounds according to the invention can be prepared by conventional methods, particularly as follows:
(A) A suitable process for the preparation of the compounds of general formula I comprises the reaction of a compound of formula II: 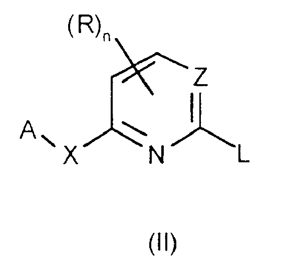 in which A, R, X, Z and n have the meaning given and L is a leaving group, with a compound of general formula 111, 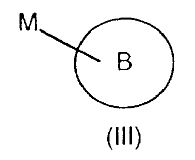 in which B has the meaning given, and
   M represents a free or complexed metal selected from the group consisting of Li, Mg, Zn, B, Sn, , in particular Li, MgHal, or B(OH)₂,
   preferably under the conditions of a cross coupling reaction.
   Suitable leaving groups L are e.g. alkyl- and arylsulfonyl, alkyl- and arylsulfonyloxy, nitro, halogen, particularly fluorine, chlorine and bromine groups.
   The cross coupling reaction generally may be carried out in the presence of a transition metal complex, as for example described in Tetrahedron **48** (1992) 8117, and Chem. Scr. **26** (1986) 305. Preferred transition metals are Pd or Ni. Compounds of general formula III may be prepared and isolated separately or may be prepared in situ.
(B) Alternatively a compound of formula IV: 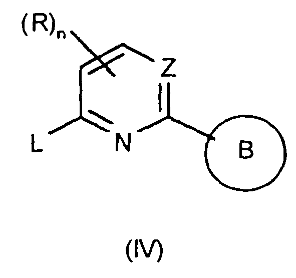 with a compound of general formula V

   A-XM¹ (V)
wherein
A, B, R, X, Z and n are defined as in Claims 1 to 6;
L represents a suitable leaving group; and
M¹ represents a metal atom.

The preferred compounds of formula IA, wherein Z denotes N may be prepared according to the following reaction scheme:

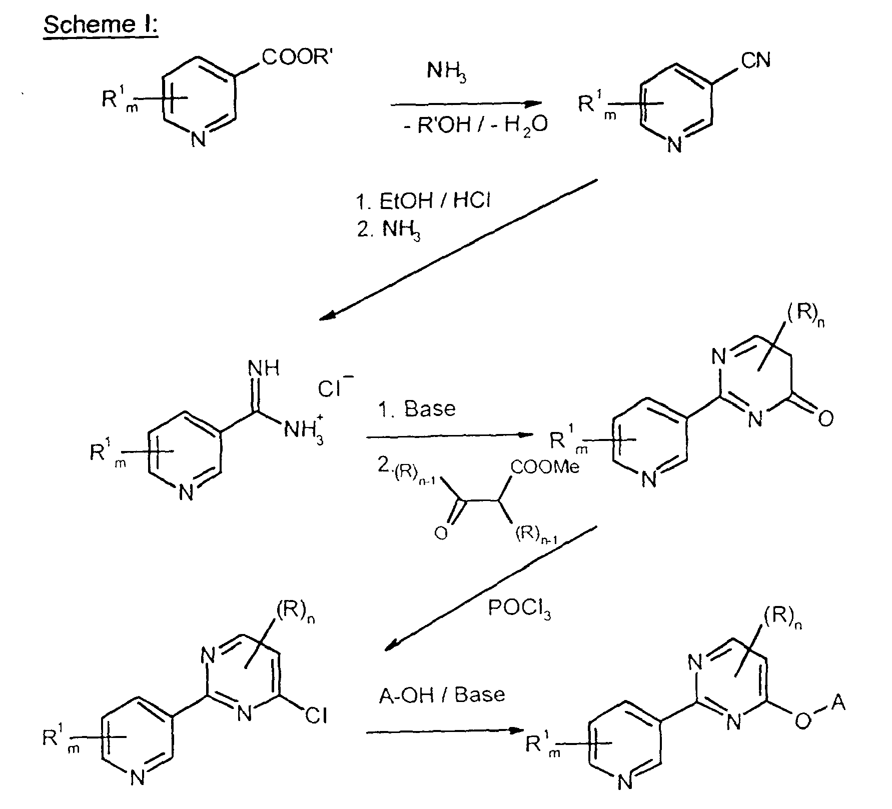

R' represents a hydrogen atom or a lower alkyl group, preferably methyl or ethyl.

The reactions according to (A) and (B) or according to scheme I may be carried out in the absence or presence of a solvent which promotes the reaction or at least does not interfere with it. Preferred are polar, aprotic or protic solvents, suitably being N,N-dimethylformamide, dimethylsulfoxide, sulfolane, acetonitrile, methyl ethyl ketone, or an ether, such as tetrahydrofurane or dioxane, or alcoholes, or water, or mixtures thereof. The reaction is carried out at a temperature between ambient temperature and the reflux temperature of the reaction mixture, preferably at elevated temperature, especially at reflux temperature.

The reactions may be carried out in the presence of a basic compound such as an alkali hydroxide, bicarbonate or carbonate, e. g. sodium or potassium hydroxide, bicarbonate or carbonate, an alkali alkoxide, e. g. sodium ethoxide, or an organic base such as triethylamine.

A hydroxy compound used in the above reactions may be present in form of a salt, preferably as a salt of an alkali metal, particularly of sodium or potassium. The presence of a copper salt may be suitable.

Suitable leaving groups L are e.g. alkyl- and arylsulfonyl, alkyl- and arylsulfonyloxy, perfluoroalkylsulfonyloxy, nitro and halogen, particularly fluorine, chlorine and bromine groups.

For compounds of formula II or IV, certain substituents R like alkyl, alkoxy, alkylthio, alkylamino, dialkylamino, amino or halo, can be introduced onto the pyridine ring by displacement of a alkyl- or arylsulfonyl, alkyl- or aryisuffonyloxy, nitro, or halogen group, or of a aryl- or hetaryloxy group like A-○ group, wherein A has the meaning given. Halogen atoms may also be introduced by diazotization of an amino group.

The compounds used as starting material are partly known and partly novel. The invention relates to the novel intermediates, in particular to the compounds of formula IV, which can be prepared analogously to known methods.

Intermediates of formula 11 and IV can suitably be prepared from compounds of formula VI, 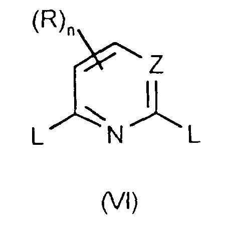 in which R, L and n have the meaning given above, by conventional methods known in pyridine chemistry, as described in : G.R.Newkome, "Pyridine and its Derivatives", in The Chemistry of Heterocyclic Compounds, Vol.14, Part 5, Eds. A. Weissberger and E.C. Taylor, John Wiley & Sons, New York - Chichester - Brisbane - Toronto - Singapore 1984.

For the preparation of the intermediates of formula II the compound of formula VI is reacted with a compound of formula V essentially under the same conditions given for method (B).

For the preparation of the intermediates of formula IV the compound of formula VI is reacted with a compound of formula III essentially under the same conditions given for method (A).

The present invention also provides the use of the compounds of formula I as herbicides. Further, in accordance with the invention there is provided a method of combating undesired plant growth at a locus by treating the locus with a composition according to the invention or an effective amount of a compound of formula I. As a useful action is by foliar spray application, the locus is most suitably the plants in a crop area, typical crops being cereals, maize, soya bean, sunflower or cotton. However, application may also be to the soil for those compounds having pre-emergence herbicidal action, or to the water of paddy rice fields. The dosage of active ingredient used may, for example be in the range of from 0.005 to 3 kg/ha, preferably 0.01 to 1 kg/ha.

The compounds of general formula I have been found to show interesting activity as herbicides. Accordingly, the invention further provides a herbicidal composition comprising a compound of formula I as defined above in association with at least one carrier, and a method of making such a composition which comprises bringing a compound of formula I into association with at least one carrier. Preferably, there are at least two carriers, at least one of which is a surface-active agent.

The invention also provides a method of combating undesired plant growth at a locus, comprising application of such a compound or composition.

Particularly interesting activity has been found against grasses and broad leaf weeds, pre- and post-emergence. Selectivity in important crop species such as wheat, barley, maize, rice and soya-beans has also been found. This activity provides a further aspect of the present invention.

In a method as mentioned above, the dosage of the active ingredient, the compound of general formula I, may, for example, be from 0.005 to 10 kg/ha, suitably 0.01 to 4 kg/ha. The locus may be an agricultural or horticultural locus, comprising, for example, a plant or soil. In a preferred method the locus contains undesired plant growth and treatment is by foliar spray application.

The invention also provides the use of a compound as defined above, as a herbicide. A carrier in a composition according to the invention is any material with which the active ingredient is formulated to facilitate application to the locus to be treated, or to facilitate storage, transport or handling. A carrier may be a solid or a liquid, including a material which is normally gaseous but which has been compressed to form a liquid, and any of the carriers normally used in formulating pesticidal compositions may be used. Preferably, compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosene and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus, preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be non-ionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkaryl sulphonates such as dodecylbenzene sulphonate; and polymers of ethylene oxide and copolymers of ethylene oxide and propylene oxide.

The compositions of the new invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing 0.5 -10% w of active ingredient. Granules are usually prepared to have a particle size between 10 and 100 BS mesh (1.676 - 0.152 mm), and may be manufactured by agglomeration or impregnation techniques. Generally, granules will contain 0.5-75% w active ingredient and 0-10% w of additives such as stabilisers, surfactants, slow release modifiers and binding agents. The so-called 'dry flowable powders' consist of relatively small granules having a relatively higher concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as antifreeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The active ingredients according to the invention can be employed alone or as formulations in combination with conventional herbicides. Such combinations of at least two herbicides can be included in the formulation or also added in a suitable form with the preparation of the tank mix. For such mixtures at least one of the following known herbicides can be used:
ametrydione, metabenzthiazuron, metamitron, metribuzin, 2,4-D, 2,4-DB, 2,4-DP, alachlor, alloxydim, asulam, atrazin, bensulfuron, bentazon, bifenox, bromoxynil, butachlor, chloridazon, chlorimuron, chlorpropham, chlorsulfuron, chlortoluron, cinmethylin, clopyralid, cyanazin, cycloate, cycloxydim, dichlobenil, diclofop, eptame, ethiozin, fenoxaprop, fluazifop, fluometuron, fluridone, fluroxypyr, fomesafen, glyphosate, haloxyfop, hexazinone, imazamethabenz, imazapyr, imazaquin, imazethapyr, ioxynil, isoproturon, lactofen, MCPA, MCPP, mefenacet, metazachlor, metolachlor, metsulfuron, molinate, norflurazon, oryzalin, oxyfluorfen, pendimethalin, picloram, pretilachlor, propachlor, pyridate, quizalofopethyl, sethoxydim, simetryne, terbutryne, thiobencarb, triallate, trifluralin, diflufenican, propanil, triclopyr, dicamba, desmedipham, acetochlor, fluoroglycofen, halosafen, tralkoxydim, amidosulfuron, cinosulfuron, nicosulfuron, pyrazosulfuron, thiameturon, thifensulfuron, triasulfuron, oxasulfuron, azimsulfuron, tribenuron, esprocarb, prosulfocarb, terbutylazin, benfuresate, clomazone, di-methazone, dithiopyr, isoxaben, quinchlorac, qinmerac, sulfosate, cyclosulfamuron, imazamox, imazamethapyr, flamprop-M-methyl, flamprop-M-isopropyl, picolinafen, fluthiamid, isoxaflutole, flurtamone, daimuron, bromobutide, methyldimron, dimethenamid, sulcotrione, sulfentrazone, oxadiargyl, acifluorfen, cafenstrole, carfentrazone, diuron, glufosinate.

Mixtures with other active ingredients like fungicides, insecticides, acaricides and nematicides are possible.

A suitable concentrated formulation containing a compound according to the invention can, for example, consist of 100 g of active ingredient (compound of formula I), 30 g of dispersing agent, 3 g of antifoaming agent, 2 g of structure agent, 50 g of anti-freezing agent, 0.5 g of a biocidal agent and water ad 1000 ml. Prior to use, it is diluted with water to give the desired concentration of active ingredient.

For a more clear understanding of the invention, specific examples are set forth below. These examples are merely illustrations and are not to be understood as limiting the scope and underlying principles of the invention in any way. Various modifications of the invention in addition to those shown and described herein will become apparent to those skilled in the art from the following examples and foregoing description. Such modifications are also intended to fall within the scope of the appended claims.

The structures of the compounds prepared in the following examples were additionally confirmed by NMR and mass spectrometry.

### Example 1

### Preparation of 2-(6-chloro-3-pyridyl)-6-methyl-4-(1-methyl-3-trifluoromethylpyrazol-5-yloxy)pyrimidine

### 1A 4-chloro-2-(6-chloro-3-pyridyl)-6-methylpyrimidine

0.92 g (5 mmol) N,N-dimethyl-6-chloro-pyridine-3-carboxamide and 0.42 g ( 5 mmol) E/Z-3-aminocrotonitrile in 15 ml POCl₃ were heated to 100°C for three hours. After cooling, the mixture is carefully quenched into water and extracted with ethyl acetate. Purification by flash chromatography yields 0.37 g of m.p. 162-164°C.

### 1B 2-(6-Chloro-3-pyridyl)-6-methyl-4-(1-methyl-3-trifluoromethylpyrazol-5-yloxy)pyrimidine

0.17 g 1A (0.71 mmol), 0.13 g (0.77 mmol) 1-methyl-3-trifluoromethylpyrazolin-5-one and 0.2 g potassium carbonate in 25 ml acetonitrile were heated to reflux for 18 hrs. The solvent was evaporated, the residue dissolved in ethyl acetate and washed with water. Drying and evaporation yields 0.26 g of m.p. 97-100°C.

### Example 2

### Preparation of 2-(6-trifluoromethyl-3-pyridyl)-5-methyl-4-(1-methyl-3-trifluoromethylpyrazol-5-yloxy) pyrimidine

### 2A Methyl 6-trifluoromethylpyridine-3-carboxylate

2.85 g (10 mmol) methyl 2-bromo-6-trifluoromethyl-pyridine-3-carboxylate is hydrogenated in 30 ml methanol and 1.5 ml triethylamine at room temperature under a hydrogen pressure of 60 psi with a 10% palladium on charcoal catalyst. After filtration and evaporation, the residue is dissolved in ethyl acetate and washed with water. Evaporation yields 1.8 g of m.p. 58-60°C.

### 2B 6-Trifluoromethylpyridine-3-carbonitrile

1 g 2A (5 mmol) in 50 ml xylene were treated with 10.5 ml of a 1.2 M solution of Me₂Al-NH₂ in toluene / dichloromethane 2:1 . The mixture was heated to reflux for 3 hrs. The cooled solution is washed with water, dried and the solvent removed by distillation. Yield 0.4 g of m.p. 34-37°C.

### 2C 6-Trifluoromethylpyridine-3-carboxamidine x HCl

4.2 g 2B (24.4. mmol) in 10 ml ethanol and 35 ml diethyl ether are cooled to 0-5°C and saturated with gaseous HCl. After stirring over night at room temperature, the mixture is evaporated and dissolved again in 35 ml ethanol. With ice cooling, an excess of gaseous ammonia is introduced, and stirring is continued over night at room temperature. The mixture is filtered, and the filtrate evaporated to give 4.5 g of m.p. 140-144°C.

### 2D 2-(6-Trifluoromethyl-3-pyridyl)-5-methyl-4-pyrimidone

2.7 g 2C (12 mmol) in 25 ml methanol are treated with 6 ml of a 25% solution of potassium methoxide in methanol at room temperature. 1.53 g methyl 2-formylpropionate (13 mmol) in 5 ml methanol are added dropwise and the mixture is stirred at room temperature over night. The solvent is evaporated, the residue dissolved in water and the product precipitated by the addition of aqueous HCl.
Yield 2.9 g of m.p. 260-265°C.

### 2E 4-Chloro-2-(6-Trifluoromethyl-3-pyridyl)-5-methylpyrimidine

2.9 g 2D and 0.1 ml N,N-dimethylanilin in 25 ml POCl₃ are heated to 100°C for 3 hrs. The mixture is carefully added into water and extracted with ethyl acetate. Purification by flash chromatography yields 1.15 g of m.p. 142-145°C.

### 2F 2-(6-Trifluoromethyl-3-pyridyl)-5-methyl-4-(1-methyl-3-trifluoromethylpyrazol-5-yloxy)pyrimidine

0.27 g 2E (1.0 mmol), 0.18 g (1.05 mmol) 1-methyl-3-trifluoromethylpyrazolin-5-one and 0.25 g potassium carbonate in 20 ml acetonitrile were heated to reflux for 6 hrs. The solvent was evaporated, the residue dissolved in ethyl acetate and washed with water. Drying and evaporation yields 0.4 g of m.p. 178-182°C.

### Examples 3-53:

Further Examples are prepared according to the general method of Example 1 or 2 and are listed in Table 1.

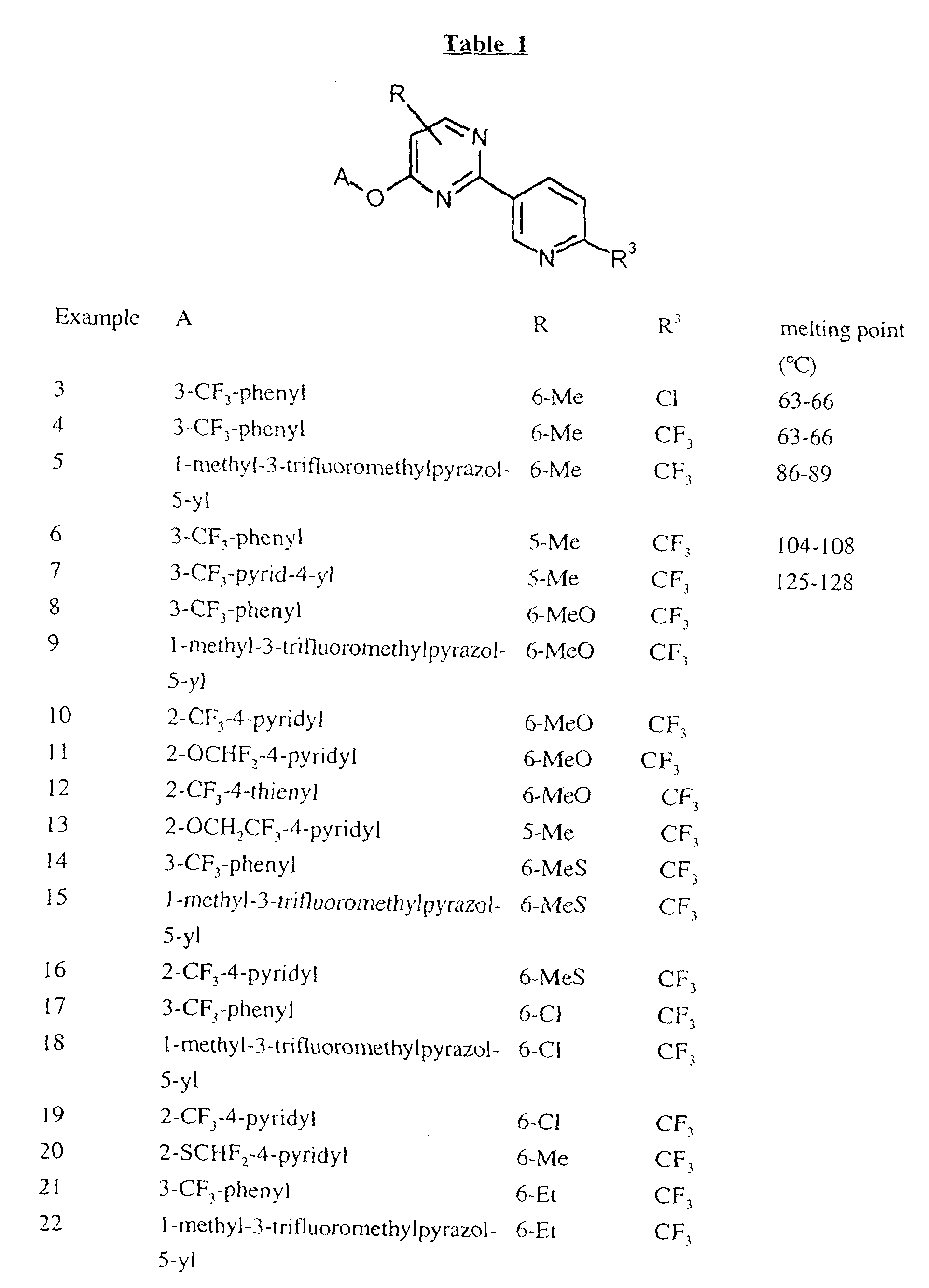

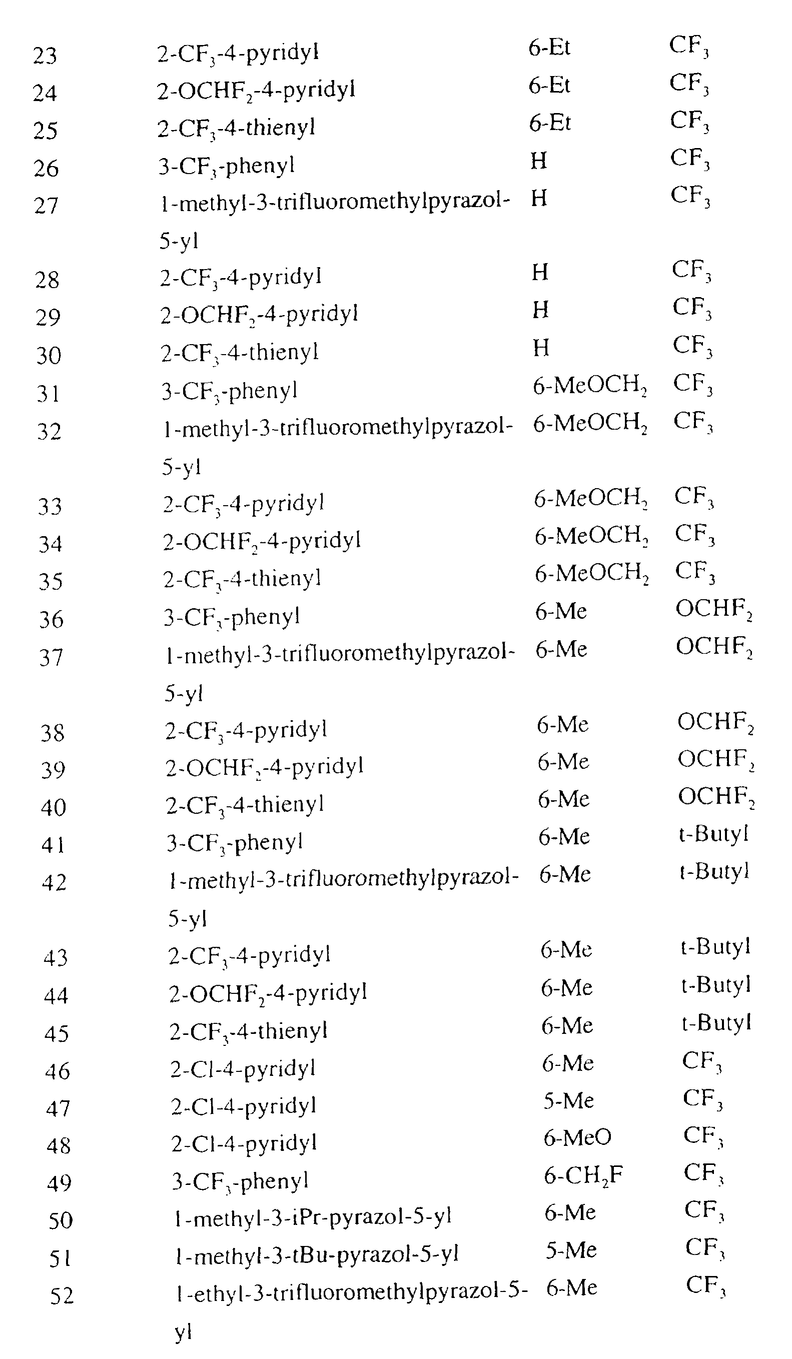

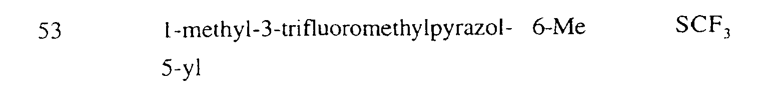

### Examples 54-63:

Further Examples are prepared according to the general method of Example 1 or 2 and are listed in Table 2.

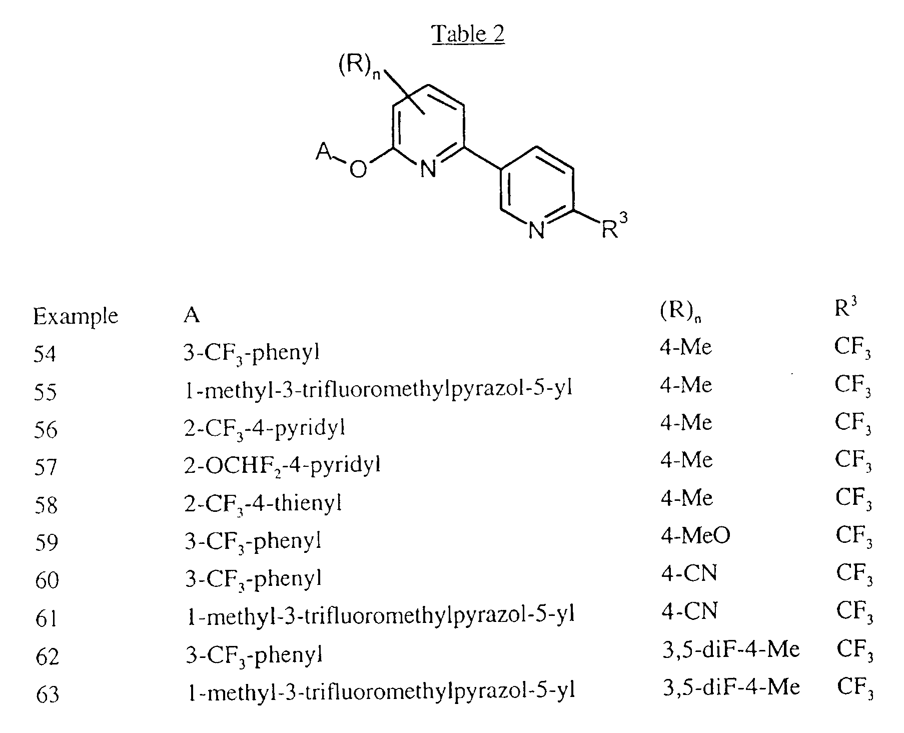

The compound of Example 54 has a melting point of 80-82 °C.

### Herbicidal Activity

To evaluate their herbicidal activity, compounds according to the invention are tested using a representative range of plants:
- TRZAW: Triticum aestivum
- ZEAMX: Zea mays
- GLXMA: Glycine max
- ALOMY: Alopecurus myosuroides
- ECHCG: Echinochloa crus-galli
- SETVI: Setaria viridis
- ABUTH: Abutilon theophrasti
- AMBEL: Ambrosia artenusifolia
- SIDSP: Sida spinosa
- IPOHE: Ipomoea hederacea

The pre-emergence tests involve spraying a liquid formulation of the compound onto the soil in which the seeds of the plant species mentioned above has recently been sown.

The soil used in the tests is a prepared horticultural loam. The formulations used in the tests are prepared from solutions of the test compounds in acetone containing 0.4% by weight of an alkylphenol/ethylene oxide condensate available under the trade mark TRITON X-155. These acetone solutions are diluted with water and the resulting formulations applied at dosage levels corresponding to 200 g, 400 g, 800 g or 1600 g of active material per hectare in a volume equivalent to 900 litres per hectare. In these tests untreated sown soil are used as controls. The herbicidal effects of the test compounds are assessed visually three weeks after spraying the soil and are recorded on a 0-9 scale. A rating 0 indicates growth as untreated control, a rating 9 indicates death. An increase of 1 unit on the linear scale approximates to a 10% increase in the level of effect. The results of the assessment are set out in Table 3.

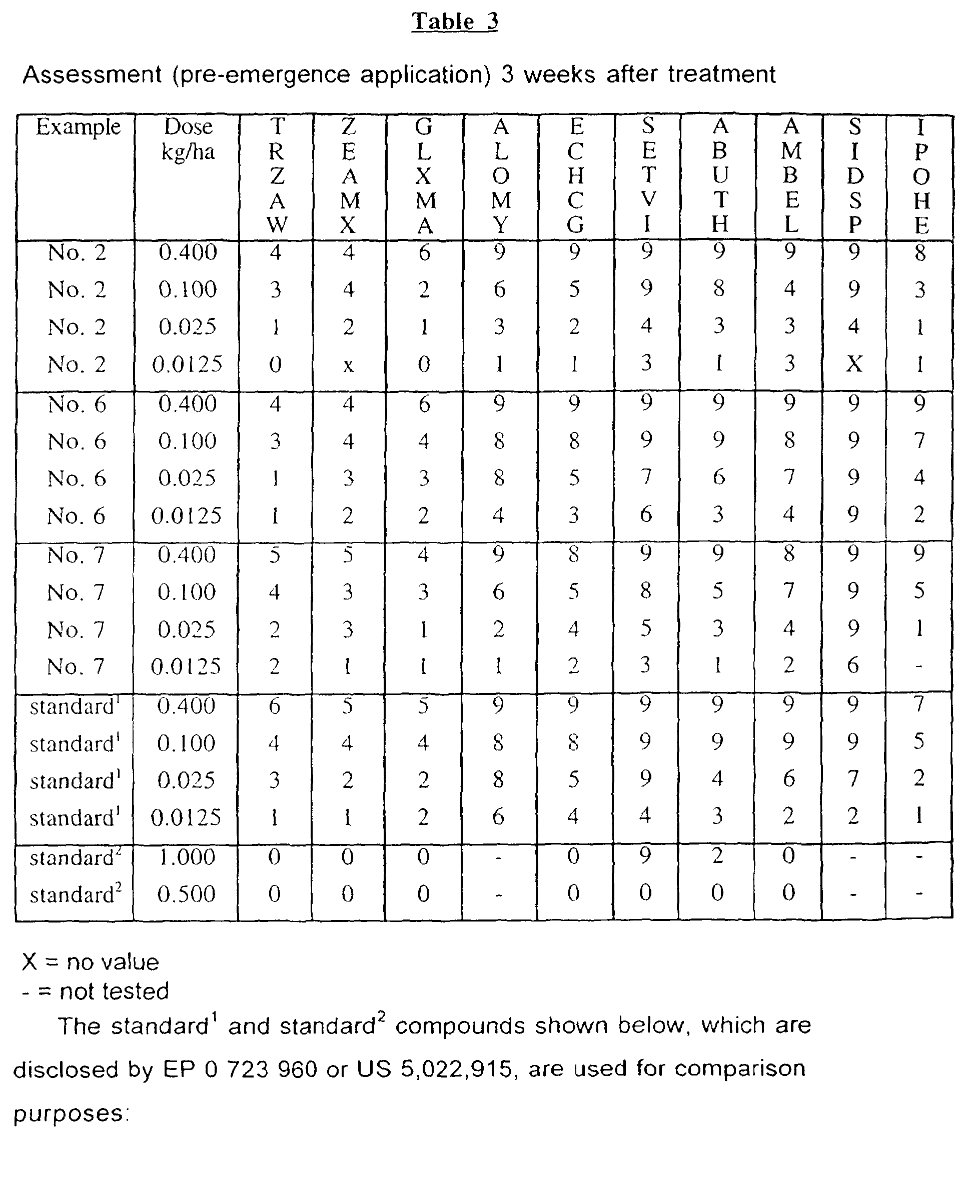

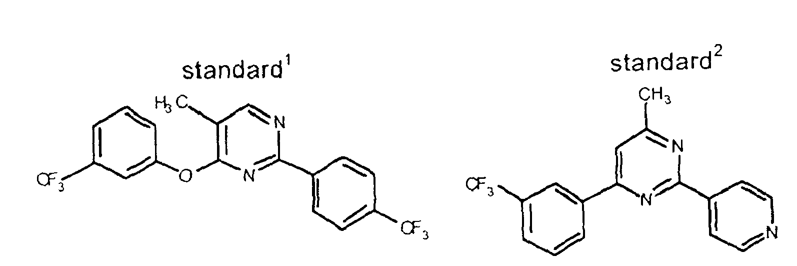

The compounds of the invention have shown to clearly improve selectivity in important crops (maize, soybeans, wheat) at doses required for good weed control when compared to the corresponding compounds of the state of the art having a 4-trifluoromethylphenyl moiety (standard') instead of the 6-trifluoromethylpyrid-3-yl group attached to a 4-aryloxypyrimidine group according to the invention. They also show improved activity, in particular against broadleaf weeds. At the dose of 25 g/ha, which was well tolerated in maize and wheat, the compounds of examples 2 and 6 demonstrated good overall levels of weed control while standard' was not sufficiently selective in these crops and standard² was almost inactive against weed at the tested dose rates.

## Claims

1. A compound of the general formula (I) 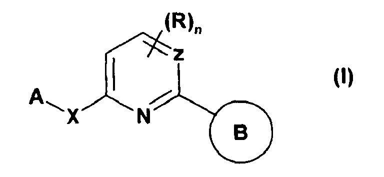 wherein
A represents a phenyl, pyridyl, pyrazolyl or thienyl group substituted by one or more of the same or different substituents selected from halogen atoms, C₁₋₄-alkyl groups, C₁₋₄-alkoxy groups, cyano groups, C₁₋₄-haloalkyl groups, C₁₋₄-haloalkoxy groups, C₁₋₄-alkylthio groups, C₁₋₄-haloalkylthio groups and SF₅ groups, and
wherein A has a substituent in the meta-position relative to the point of attachment;
B represents an optionally substituted 6-membered nitrogen-containing heteroaromatic group, in which the optional substituents are one or more, same or different, of the following: halogen, nitro, cyano, amino, hydroxy, phenoxy, C₁₋₄-alkyl, C₁₋₄alkoxy, C₁₋₄-haloalkyl, C₁₋₄-haloalkenyl, C₁₋₄-haloalkoxy, C₁₋₄-haloalkylthio, C₁₋₄-alkylsulfonyl and halosulfanyl;
R represents a halogen atom or an optionally substituted alkyl, alkenyl, alkynyl, alkoxy, alkoxyalkyl, alkoxyalkoxy group; or
a haloalkyl, haloalkoxy, cyano, nitro group; or
-S(O)ₚ-R⁵, in which p is 0, 1 or 2, and in which R⁵ represents an alkyl or a haloalkyl group; or
-NR⁶R⁷, in which R⁶ and R⁷ independently represent a hydrogen atom, an alkyl, alkenyl, aralkyl or aryl group; or
R⁸OCY-, in which R⁸ represents an alkyl group, and Y represents O or S;
in which the optional substituents for an optionally substituted alkyl group or alkyl part are selected from phenyl, halogen atoms, nitro, cyano, hydroxyl, C₁-C₄-alkoxy, C₁-C₄-haloalkoxy and C₁-C₄-alkoxycarbonyl groups;
wherein the alkyl, alkenyl and alkynyl moieties contain 1 to 6 carbon atoms; the alkyl parts of haloalkyl, haloalkoxy, alkylthio or alkoxy group contain 1 to 4 carbon atoms and the alkoxyalkyl and alkoxyalkoxy groups contain up to 6 carbon atoms;
X represents an oxygen or a sulfur atom,
Z represents CH or N, and
n represents an integer from 0 to 3,
with the provisos that
B represents an optionally substituted pyridine group, when Z denotes CH.

2. A compound as claimed in Claim 1, wherein
Z represents N, and
B represents an optionally substituted pyrid-3-yl or pyrid-4-yl group, in which the optional substituents are one or more, same or different, of the following: halogen, nitro, cyano, amino, hydroxy, phenoxy, C₁₋₄₋alkyl, C₁₋₄-alkoxy, C₁₋₄-haloalkyl, C₁₋₄-haloalkenyl, C₁₋₄-haloalkoxy, C₁₋₄₋haloalkylthio, C₁₋₄-alkylsulfonyl and halosulfanyl.

3. A compound as claimed in Claim 1, wherein A is meta-substituted by a chlorine atom, or a trifluoromethyl, trifluoromethoxy or difluoromethoxy group.

4. A compound as claimed in Claim 1, wherein B represents a pyridyl group being substituted by one or more of the same or different substituents selected from halogen atoms, alkyl groups, alkoxy groups, cyano groups, nitro groups, haloalkyl groups, haloalkoxy groups, alkylthio groups, haloalkylthio groups and SF₅ groups.

5. A compound as claimed in Claim 4, wherein B represents a pyrid-3-yl or pyrid-4-yl group being substituted in its 6-position by a substituent selected from halogen atoms and haloalkyl groups.

6. A compound as claimed in Claim 1, wherein X is oxygen.

7. A compound according to claim 1 of formula IA 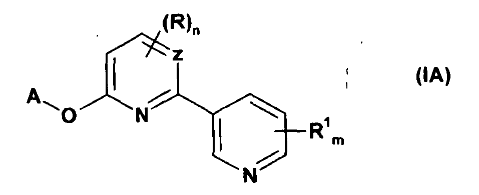 wherein
A represents 3-trifluoromethylphenyl, 2-chlorpyrid-4-yl, 2-trifluoromethylpyrid-4-yl, 2-difluoromethoxypyrid-4-yl, 2-trifluoromethylthien-4-yl or 1-methyl-3-trifluoromethylpyrazol-5-yl;
Z and R have the meaning given above;
R¹ each independently represent a hydrogen or a fluorine atom, one or two of them also a chlorine or bromine or a trifluoromethyl, difluoromethoxy or a cyano group, one of them can further be a C₁-C₄-alkyl group, particular tert-butyl, at least one of R¹ being different from hydrogen;
m is an integer selected from 1 to 4, and
n is an integer selected from 0 to 2.

8. A compound according to Claim 1 selected from the group consisting of 6-methyl-4-(1'-methyl-3'-trifluoromethylpyrazol-5'-yloxy)-2-(6"-trifluoromethyl-pyrid-3"-yl)pyrimidine,
6-methyl-4-(1'-methyl-3'-trifluoromethylpyrazol-5'-yloxy)-2-(6"-chloropyrid-3"-yl)pyrimidine,
6-methyl-4-(3'-trifluoromethylphenyloxy)-2-(6"-trifluoromethylpyrid-3"-yl)-pyrimidine,
6-methyl-4-(3'-trifluoromethylphenyloxy)-2-(6"-chloropyrid-3"-yl)pyrimidine,
5-methyl-4-(3'-trifluoromethylphenyloxy)-2-(6"-trifluoromethylpyrid-3"-yl)-pyrimidine,
5-methyl-4-(1'-methyl-3'-trifluoromethylpyrazol-5'-yloxy)-2-(6"-trifluoromethylpyrid-3"-yl)pyrimidine, and
5-methyl-4-(6'-trifluoromethylpyrid-3-yloxy)-2-(6"-trifluoromethylpyrid-3"-yl)pyrimidine.

9. A process for the preparation of a compound of formula I as defined in claim 1, which comprises reacting a respective compound of the general formula II, 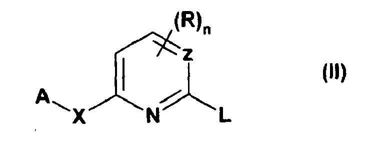 in which A, R, X, Z and n have the same meaning as in formula (I) and L is a leaving group selected from the group consisting of alkyl- and arylsulfonyl, alkyl- and arylsulfonyloxy, nitro and halogen groups, with a compound of general formula III, 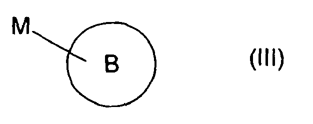 in which B has the same meaning as in formula I, and
M represents a free or complexed metal selected from the group consisting of Li, Mg, Zn, B, Sn, optionally in the presence of a transistion metal complex.

10. A process for the preparation of a compound of formula I as defined in Claim 1, which comprises reacting a respective compound of the general formula IV, 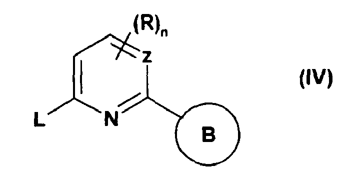 with a compound of general formula V
A-XM¹ (V)
wherein A, B, R, X, Z and n are defined as in Claims 1;
L represents a leaving group selected from the group consisting of alkyl- and arylsulfonyl, alkyl- and arylsulfonyloxy, nitro and halogen groups, and
M¹ represents a metal atom.

11. A compound of formula IV 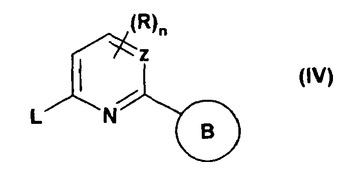 wherein B, R, Z and n are defined as in Claim 1;
L represents a leaving group as defined in Claim 10.

12. A herbicidal composition comprising at least one compound of general formula I, as claimed in Claim 1, together with an agronomically acceptable carrier.

13. A composition as claimed in Claim 12, comprising at least two carriers, at least one of which is a surface-active agent.

14. A method of combating undesired plant growth at a locus, comprising application to the locus a herbicidally effective amount of a compound of general formula I, as claimed in Claim 1.

## Patentansprüche

1. Verbindung der allgemeinen Formel (I), 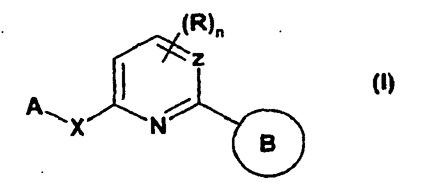 in der
A eine Phenyl-, Pyridyl-, Pyrazolyl- oder Thienylgruppe, die durch einen oder mehrere gleiche oder verschiedene Substituenten aus der Reihe Halogenatome, C₁₋₄-Alkyl-Gruppen, C₁₋₄-Alkoxy-Gruppen, Cyanogruppen, C₁₋₄₋Halogenalkyl-Gruppen, C₁₋₄-Halogenalkoxy-Gruppen, C₁₋₄-Alkylthio-Gruppen, C₁₋₄₋Halogenalkylthio-Gruppen und SF₅-Gruppen, substituiert ist, und wobei A einen Substituenten in meta-Stellung in bezug auf die Bindungsstelle aufweist;
B eine gegebenenfalls substituierte 6-gliedrige stickstoffhaltige heteroaromatische Gruppe, in der die gegebenenfalls vorhandenen Substituenten einen oder mehrere, gleiche oder verschiedene Substituenten aus der folgenden Gruppe: Halogen, Nitro, Cyano, Amino, Hydroxy, Phenoxy, C₁₋₄-Alkyl, C₁₋₄-Alkoxy, C₁₋₄-Halogenalkyl, C₁₋₄-Halogenalkenyl, C₁₋₄-Halogenalkoxy, C₁₋₄-Halogenalkylthio, C₁₋₄-Alkylsulfonyl und Halogensulfanyl sind, bedeutet;
R ein Halogenatom oder eine gegebenenfalls substituierte Alkyl-, Alkenyl-, Alkinyl-, Alkoxy-, Alkoxyalkyl-, Alkoxyalkoxygruppe; oder
eine Halogenalkyl-, Halogenalkoxy-, Cyano-, Nitrogruppe; oder -S(O)ₚ-R⁵, worin p 0, 1 oder 2 bedeutet und worin R⁵ eine Alkyl- oder Halogenalkylgruppe bedeutet; oder
-NR⁶R⁷, worin R⁶ und R⁷ unabhängig ein Wasserstoffatom, eine Alkyl-, Alkenyl-, Aralkyl- oder Arylgruppe bedeuten; oder R⁸OCY-, worin R⁸ eine Alkylgruppe bedeutet und Y O oder S bedeutet;
worin die gegebenenfalls vorhandenen Substituenten für eine gegebenenfalls substituierte Alkylgruppe oder einen gegebenenfalls substituierten Alkylteil aus der Reihe Phenyl, Halogenatome, Nitro, Cyano, Hydroxy, C₁₋₄-Alkoxy, C₁₋₄-Halogenalkoxy und C₁₋₄-Alkoxycarbonylgruppen stammen;
wobei die Alkyl-, Alkenyl- und Alkinylreste 1 bis 6 Kohlenstoffatome enthalten, die Alkylteile einer Halogenalkyl-, Halogenalkoxy-, Alkylthio- oder Alkoxygruppe 1 bis 4 Kohlenstoffatome enthalten und die Alkoxyalkyl- und Alkoxyalkoxygruppen bis zu 6 Kohlenstoffatome enthalten, bedeutet;
X ein Sauerstoff- oder ein Schwefelatom bedeutet,
Z CH oder N bedeutet, und
n eine ganze Zahl von 0 bis 3 bedeutet,
mit den Maßgaben, daß, wenn Z CH bedeutet,
B eine gegebenenfalls substituierte Pyridingruppe bedeutet.

2. Verbindung nach Anspruch 1, in der
Z N bedeutet, und
B eine gegebenenfalls substituierte Pyrid-3-yl- oder Pyrid-4-ylgruppe, in der die gegebenenfalls vorhandenen Substituenten ein oder mehrere, gleiche oder verschiedene Substituenten aus der folgenden Reihe: Halogen, Nitro, Cyano, Amino, Hydroxy, Phenoxy, C₁₋₄-Alkyl, C₁₋₄-Alkoxy, C₁₋₄-Halogenalkyl, C₁₋₄-Halogenalkenyl, C₁₋₄-Halogenalkoxy, C₁₋₄-Halogenalkylthio, C₁₋₄-Alkylsulfonyl und Halogensulfanyl sind.

3. Verbindung nach Anspruch 1, in der A durch ein Chloratom oder eine Trifluormethyl-, Trifluormethoxy- oder Difluormethoxygruppe metasubstituiert ist.

4. Verbindung nach Anspruch 1, in der B eine Pyridylgruppe, die durch einen oder mehrere gleiche oder verschiedene Substituenten aus der Reihe Halogenatome, Alkylgruppen, Alkoxygruppen, Cyanogruppen, Nitrogruppen, Halogenalkylgruppen, Halogenalkoxygruppen, Alkylthiogruppen, Halogenalkylthiogruppen und SF₅-Gruppen, substituiert ist, bedeutet.

5. Verbindung nach Anspruch 4, in der B eine Pyrid-3-yl- oder Pyrid-4-ylgruppe, die in 6-Stellung durch einen Substituenten aus der Reihe Halogenatome und Halogenalkylgruppen substituiert ist, bedeutet.

6. Verbindung nach Anspruch 1, in der X Sauerstoff bedeutet.

7. Verbindung nach Anspruch 1, der Formel IA, 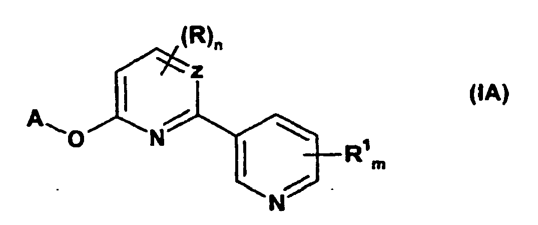 in der
A 3-Trifluormethylphenyl, 2-Chlorpyrid-4-yl, 2-Trifluormethylpyrid-4-yl, 2-Difluormethoxypyrid-4-yl, 2-Trifluormethylthien-4-yl oder 1-Methyl-3-trifluormethylpyrazol-5-yl bedeutet;
Z und R die oben genannte Bedeutung aufweisen;
R¹ jeweils unabhängig ein Wasserstoff- oder ein Fluoratom bedeutet, einer oder zwei dieser Reste auch eine Chlor- oder Brom- oder eine Trifluormethyl-, Difluormethoxy- oder eine Cyanogruppe bedeuten können, einer dieser Reste weiterhin eine C₁₋₄-Alkylgruppe, insbesondere tert.-Butyl, bedeuten kann, wobei mindestens einer der Reste R¹ nicht Wasserstoff ist;
m eine ganze Zahl von 1 bis 4 bedeutet, und
n eine ganze Zahl von 0 bis 2 bedeutet.

8. Verbindung nach Anspruch 1 aus der Gruppe
6-Methyl-4-(1'-methyl-3'-trifluormethylpyrazol-5'-yloxy)-2-(6"-trifluormethylpyrid-3"-yl)pyrimidin,
6-Methyl-4-(1'-methyl-3'-trifluormethylpyrazol-5'-yloxy)-2-(6"-chlorpyrid-3"-yl)pyrimidin,
6-Methyl-4-(3'-trifluormethylphenyloxy)-2-(6"-trifluormethylpyrid-3"-yl)pyrimidin,
6-Methyl-4-(3'-trifluormethylphenyloxy)-2-(6"-chlorpyrid-3"-yl)pyrimidin,
5-Methyl-4-(3'-trifluormethylphenyloxy)-2-(6"-trifluormethylpyrid-3"-yl)pyrimidin,
5-Methyl-4-(1'-methyl-3'-trifluormethylpyrazol-5'-yloxy)-2-(6"-trifluormethylpyrid-3"-yl)pyrimidin und
5-Methyl-4-(6'-trifluormethylpyrid-3-yloxy)-2-(6"-trifluormethylpyrid-3"-yl)pyrimidin.

9. Verfahren zur Herstellung einer Verbindung der Formel I nach Anspruch 1, bei dem man eine entsprechende Verbindung der allgemeinen Formel II, 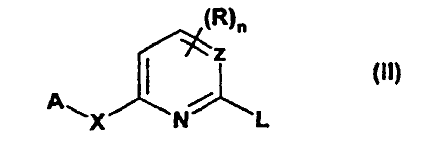 in der A, R, X, Z und n die gleiche Bedeutung wie in Formel (I) aufweisen und L eine Abgangsgruppe aus der Reihe Alkyl- und Arylsulfonyl-, Alkyl- und Arylsulfonyloxy-, Nitro- und Halogengruppen bedeutet, mit einer Verbindung der allgemeinen Formel III, 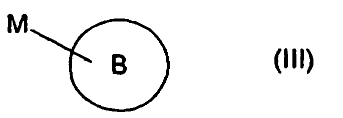 in der B die gleiche Bedeutung wie in Formel I aufweist und
M ein freies oder komplexiertes Metall aus der Gruppe Li, Mg, Zn, B, Sn bedeutet, gegebenenfalls in Gegenwart eines Übergangsmetallkomplexes umsetzt.

10. Verfahren zur Herstellung einer Verbindung der Formel I nach Anspruch 1, bei dem man eine entsprechende Verbindung der allgemeinen Formel IV, 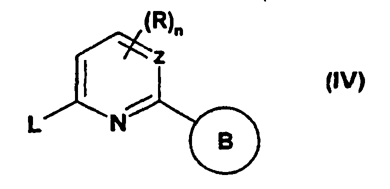 mit einer Verbindung der allgemeinen Formel V
**A-XM**^{**1**} **(V)**
in der A, B, R, X, Z und n wie in Anspruch 1 definiert sind,
L eine Gruppe aus der Reihe Alkyl- und Arylsulfonyl-, Alkyl- und Arylsulfonyloxy-, Nitro- und Halogengruppen bedeutet, und
M¹ ein Metallatom bedeutet,
umsetzt.

11. Verbindung der Formel IV, 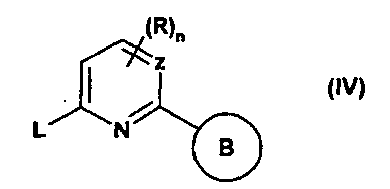 in der B, R, Z und n wie in Anspruch 1 definiert sind;
L eine wie in Anspruch 10 definierte Abgangsgruppe bedeutet.

12. Herbizide Zusammensetzung, die mindestens eine Verbindung der allgemeinen Formel I nach Anspruch 1 gemeinsam mit einem landwirtschaftlich annehmbaren Träger umfaßt.

13. Zusammensetzung nach Anspruch 12, die mindestens zwei Träger, von denen mindestens einer ein Tensid ist, umfaßt.

14. Verfahren zur Bekämpfung von unerwünschtem Pflanzenwachstum an einem Ort, bei dem man eine herbizidwirksame Menge einer Verbindung der allgemeinen Formel I nach Anspruch 1 auf den Ort ausbringt.

## Revendications

1. Composé de la formule générale (I) : 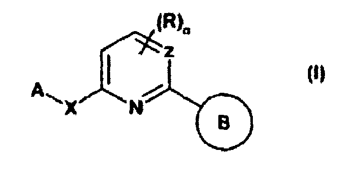 dans laquelle
A représente un groupe phényle, pyridyle, pyrazolyle ou thiényle substitué par un ou plusieurs substituants identiques ou différents choisis parmi des atomes d'halogène, des groupes alkyle en C₁-C₄, alcoxy en C₁-C₄, cyano, halogénoalkyle en C₁-C₄, halogénoalcoxy en C₁-C₄, alkylthio en C₁-C₄, halogénoalkylthio en C₁-C₄ et SF₅,
A comportant un substituant dans la position méta par rapport au point de liaison,
B représente un groupe hétéroaromatique hexagonal contenant de l'azote, éventuellement substitué, dans lequel les substituants éventuels consistent en un ou plusieurs des substituants suivants, identiques ou différents : halogène, nitro, cyano, amino, hydroxy, phénoxy, alkyle en C₁-C₄, alcoxy en C₁-C₄, halogénoalkyle en C₁-C₄, halogénoalcényle en C₁-C₄, halogénoalcoxy en C₁-C₄, halogénoalkylthio en C₁-C₄, alkylsulfonyle en C₁-C₄ et halogénosulfanyle,
R représente un atome d'halogène ou un groupe éventuellement substitué alkyle, alcényle, alcynyle, alcoxy, alcoxyalkyle, alcoxyalcoxy, ou
un groupe halogénoalkyle, halogénoalcoxy, cyano, nitro, ou
-S(O)ₚ-R⁵, où p vaut 0, 1 ou 2 et R⁵ représente un groupe alkyle ou halogénoalkyle, ou
-NR⁶R⁷, où R⁶ et R⁷ représentent indépendamment un atome d'hydrogène, un groupe alkyle, alcényle, aralkyle ou aryle, ou
R⁸OCY-, où R⁸ représente un groupe alkyle et Y représente O ou S,
les substituants éventuels pour une partie alkyle ou un groupe alkyle éventuellement substitué étant choisis parmi des atomes d'halogène et des groupes phényle, nitro, cyano, hydroxyle, alcoxy en C₁-C₄, halogénoalcoxy en C₁-C₄ et alcoxycarbonyle en C₁-C₄,
les fragments alkyle, alcényle et alcynyle contenant 1 à 6 atomes de carbone, les parties alkyle d'un groupe halogénoalkyle, halogénoalcoxy, alkylthio ou alcoxy contenant 1 à 4 atomes de carbone et les groupes alcoxyalkyle et alcoxyalcoxy contenant jusqu'à 6 atomes de carbone,
X représente un atome d'oxygène ou de soufre,
Z représente CH ou N, et
n représente un nombre entier de 0 à 3,
à la condition que
B représente un groupe pyridine éventuellement substitué, lorsque Z représente CH.

2. Composé suivant la revendication 1, dans lequel
Z représente N, et
B représente un groupe pyrid-3-yle ou pyrid-4-yle éventuellement substitué, dans lequel les substituants éventuels consistent en un ou plusieurs des substituants suivants, identiques ou différents : halogène, nitro, cyano, amino, hydroxy, phénoxy, alkyle en C₁-C₄, alcoxy en C₁-C₄, halogénoalkyle en C₁-C₄, halogénoalcényle en C₁-C₄, halogénoalcoxy en C₁-C₄, halogénoalkylthio en C₁-C₄, alkylsulfonyle en C₁-C₄ et halogénosulfanyle.

3. Composé suivant la revendication 1, dans lequel A est substitué en position méta par un atome de chlore ou un groupe trifluorométhyle, trifluorométhoxy ou difluorométhoxy.

4. Composé suivant la revendication 1, dans lequel B représente un groupe pyridyle qui est substitué par un ou plusieurs substituants identiques ou différents choisis parmi des atomes d'halogène et des groupes alkyle, alcoxy, cyano, nitro, halogénoalkyle, halogénoalcoxy, alkylthio, halogénoalkylthio et SF₅.

5. Composé suivant la revendication 4, dans lequel B représente un groupe pyrid-3-yle ou pyrid-4-yle qui est substitué dans sa position 6 par un substituant choisi parmi des atomes d'halogène et des groupes halogénoalkyle.

6. Composé suivant la revendication 1, dans lequel X est de l'oxygène.

7. Composé suivant la revendication 1, répondant à la formule IA : 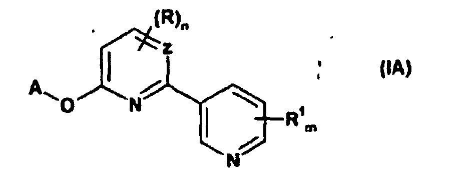 dans laquelle
A représente un groupe 3-trifluorométhylphényle, 2-chloropyrid-4-yle, 2-trifluorométhylpyrid-4-yle, 2-difluorométhoxypyrid-4-yle, 2-trifluorométhylthién-4-yle ou 1-méthyl-3- trifluorométhylpyrazol-5-yle,
Z et R ont la signification donnée précédemment,
les R¹ représentent chacun indépendamment un atome d'hydrogène ou de fluor, un ou deux d'entre eux représentant également un atome de chlore ou de brome ou un groupe trifluorométhyle, difluorométhoxy ou cyano, l'un d'eux pouvant en outre être un groupe alkyle en C₁-C₄, en particulier tert-butyle, au moins un des R¹ étant différent de l'hydrogène,
m est un nombre entier choisi entre 1 et 4, et
n est un nombre entier choisi entre 0 et 2.

8. Composé suivant la revendication 1, choisi parmi le groupe constitué
de la 6-méthyl-4-(l'-méthyl-3'-trifluorométhylpyrazol-5'-yloxy)-2-(6"-trifluorométhylpyrid-3"-yl)pyrimidine,
de la 6-méthyl-4-(1'-méthyl-3'-trifluorométhylpyrazol-5'-yloxy)-2-(6"-chloropyrid-3"-yl)pyrimidine,
de la 6-méthyl-4-(3'-trifluorométhylphényloxy)-2-(6"-trifluorométhylpyrid-3"-yl)pyrimidine,
de la 6-méthyl-4-(3'-trifluorométhylphényloxy)-2-(6"-chloropyrid-3"-yl)pyrimidine,
de la 5-méthyl-4-(3'-trifluorométhylphényloxy)-2-(6"-trifluorométhylpyrid-3"-yl)pyrimidine,
de la 5-méthyl-4-(1'-méthyl-3'-trifluorométhylpyrazol-5'-yloxy)-2-(6"-trifluorométhylpyrid-3"-yl)pyrimidine, et de la 5-méthyl-4-(6'-trifluorométhylpyrid-3-yloxy)-2-(6"-trifluorométhylpyrid-3"-yl)pyrimidine.

9. Procédé de préparation d'un composé de formule I, tel que défini dans la revendication 1, qui comprend une réaction d'un composé respectif de la formule générale II : 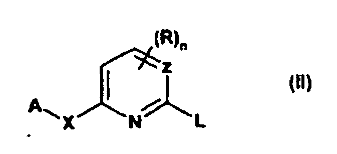 dans laquelle A, R, X, Z et n ont la même signification que dans la formule (I) et L est un groupe partant choisi parmi le groupe constitué des groupes alkylsulfonyle, arylsulfonyle, alkylsulfonyloxy, arylsulfonyloxy et nitro et des atomes d'halogène, avec un composé de formule générale III : 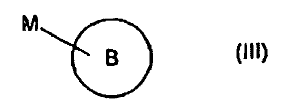 dans laquelle B a la même signification que dans la formule I, et
M représente un métal libre ou complexé choisi parmi le groupe constitué de Li, Mg, Zn, B, Sn, éventuellement en présence d'un complexe de métal de transition.

10. Procédé de préparation d'un composé de formule I, tel que défini dans la revendication 1, qui comprend une réaction d'un composé respectif de la formule générale IV : 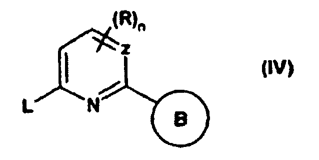 avec un composé de formule générale V :
A - XM¹ (V)
dans laquelle A, B, R, X, Z et n sont définis comme dans la revendication 1,
L représente un groupe partant choisi parmi le groupe constitué des groupes alkylsulfonyle, arylsulfonyle, alkylsulfonyloxy, arylsulfonyloxy et nitro et des atomes d'halogène, et
M¹ représente un atome de métal.

11. Composé de formule IV : 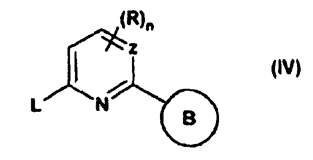 dans laquelle B, R, Z et n sont définis comme dans la revendication 1, et
L représente un groupe partant tel que défini dans la revendication 10.

12. Composition herbicide comprenant au moins un composé de formule générale I, tel que revendiqué dans la revendication 1, conjointement avec un support acceptable en agronomie.

13. Composition suivant la revendication 12, comprenant au moins deux supports, dont au moins un est un agent tensioactif.

14. Procédé pour lutter contre la croissance de plante non souhaitable en un emplacement, comprenant une application à l'emplacement d'une quantité efficace du point de vue herbicide d'un composé de formule générale I, tel que revendiqué dans la revendication 1.
